# EUROPEAN PATENT APPLICATION

(11) **EP 3 572 070 A1**
(43) Date of publication of application: **27.11.2019**
(21) Application number: 18769937.6
(22) Date of filing: 18.04.2018
(51) Int. Cl.: A61K 9/127, A61K 47/12, A61K 47/14, A61K 31/445, A61K 31/167, A61P 23/02

(54) **LOCAL ANESTHESIA PAIN-RELIEVING AND SUSTAINED-RELEASE DRUG DELIVERY SYSTEM, AS WELL AS PREPARATION METHOD THEREFOR AND USE THEREOF**

(30) Priority: 11.04.2018 CN 201810320900
(71) Applicant: Xi' an Libang Biomedical Technology Co., Ltd., Xian, Shaanxi 710000 (CN)
(72) Inventor: MA, Yufan, Xian, Shaanxi 710000 (CN); CHEN, Tao, Xian, Shaanxi 710000 (CN); KONG, Danfeng, Xian, Shaanxi 710000 (CN); LIU, Qian, Xian, Shaanxi 710000 (CN)
(74) Representative: Vitina, Maruta
(86) International application number: PCT/CN2018/083447
(87) International publication number: WO 2019/196129

(57) **Abstract**

The present invention discloses a novel local anesthetic analgesic sustained-release drug delivery system, including an internal aqueous phase, an external aqueous phase, an oil phase, an organic solvent, an isoosmotic regulator and a pH regulator, wherein the internal aqueous phase includes an analgesic, a drug solvent and a drug solubilizer, wherein the analgesic is selected from one of bupivacaine, levobupivacaine, ropivacaine, lidocaine and tetracaine, and the analgesic is in a free base form or an acid saline form; the drug solvent is selected from inorganic acid containing N or P; and the drug solubilizer is selected from one or more of saccharide and ring-shaped organic acid. The multivesicular liposome prepared in the present invention has the advantages of high encapsulation percentage and drug loading capacity, uniform grain size, and good sustained-release effect.

## Description

### Technical Field

The present invention relates to the technical field of medicine, and particularly to a local anesthetic analgesic sustained-release drug delivery system, and a preparation method and application thereof.

### Background

Clinical local anesthetic drugs mainly include esters and amides, the action duration of these drugs is less than 3 hours in general, and continuous administration is needed to maintain the appropriate plasma concentration in order to achieve the purpose of long-time analgesia. If such drugs are prepared into preparations with sustained-release functions such as microspheres, multivesicular liposomes and the like, the analgesic effect may be kept for a long time, and meanwhile, the tolerance of patients may be increased. Chinese patent with the patent no. CN102274183 B proposes two preparation methods of multivesicular liposomes. The first method includes: dissolving active pharmaceutical ingredient and osmotic pressure regulator in water to prepare an internal aqueous phase, adding a lipid phase prepared by dissolving lipid in an organic solvent to prepare W/O primary emulsion, adding an external aqueous phase containing osmotic pressure regulator to the upper layer of the W/O primary emulsion to prepare W/O/W multiple emulsion, and then removing the organic solvent from the multiple emulsion. In the method, coemulsifiers, which are frequently-used surfactants mainly for dextran, polyvinyl pyrrolidone and other tablets, are added in the preparation process, and the pH regulator is an acid frequently used in this technical field. The second method is different from the above-mentioned method in that: in the drug loading process thereof, a drug is dissolved in the external aqueous phase, to load the drug through the difference between the internal osmotic pressure and the external osmotic pressure of the multivesicular liposome rather than add the drug when preparing W/O. The method is similar to the pH gradient drug loading method or ion gradient drug loading method for ordinary liposomes. Because the multivesicular liposome is of a structure that most small vesicles are in a big vesicle, when drug is loaded using the technology, it is certainly that drug is preferentially loaded in the small vesicles of the outer layer. After drug is loaded in the small vesicles of the outer layer, the osmotic pressure of the small vesicles in the big vesicle after loading drug may be reduced. Thus, it is certainly that some small vesicles in the big vesicle of the multivesicular liposome are not filled with active drug. In addition, the multivesicular liposome is different from the ordinary liposome in that the multivesicular liposome contains triglyceride and other oil ingredients existing in the skeleton structure of each vesicle. In the patent, drug is dissolved in the external aqueous phase, so that the water-based drug must be disturbed by triglyceride in the process of entering the internal aqueous phase from the external aqueous phase and then the drug cannot enter inside the multivesicular liposome successfully. Moreover, the active pharmaceutical ingredients of the patent mainly include exenatide, topotecan and other drug which are unstable under the alkaline condition.

On this basis, the present invention provides a preparation of a multivesicular liposome containing an amide-type local anesthetic analgesic, thereby preparing a multivesicular liposome product with the advantages of high encapsulation percentage and good stability.

### Summary

The technical problem to be solved by the present invention is to provide a preferable multivesicular liposome and a preparation method thereof, thereby preparing a multivesicular liposome product having the advantages of high encapsulation percentage and good stability and meeting various requirements.

To achieve the above purpose, the present invention adopts the following technical solution:
A novel local anesthetic analgesic sustained-release drug delivery system, including an internal aqueous phase, an external aqueous phase, an oil phase, an organic solvent, an isoosmotic regulator and a pH regulator, wherein the internal aqueous phase includes an analgesic, a drug solvent and a drug solubilizer; the external aqueous phase is removed finally, and the isoosmotic regulator and the pH regulator are added finally to obtain the sustained-release drug delivery system of the present invention.

The analgesic is selected from one of bupivacaine, levobupivacaine, ropivacaine, lidocaine and mepivacaine.

The drug solvent is selected from inorganic acid containing N or P, preferably, nitric acid and phosphoric acid.

The drug solubilizer is selected from saccharide or ring-shaped organic acid, preferably, ring-shaped organic acid; the saccharide is selected from monosaccharide or binary saccharide such as cane sugar, dextrose, fructose and the like, and the ring-shaped organic acid is selected from vitamin C, nicotinic acid, gallic acid or glucuronic acid.

The external aqueous phase is selected from one or more of saccharide, ring-shaped organic acid, organic base and deflocculant; the organic base is selected from lysine or arginine; and the deflocculant is selected from citrate, tartrate or phosphate.

The oil phase includes synthetic phospholipid, synthetic phosphatidylglycerol, cholesterol and glyceride. The synthetic phospholipid is selected from one or more of dierucoyl phosphatidylcholine (DEPC), dioleoyl phosphatidylcholine (DOPC), dipalmitoyl phosphatidylcholine (DPPC), distearoyl phosphatidylcholine (DSPC) and dimyristoyl phosphatidylcholine (DMPC); the synthetic phosphatidylglycerol is selected from one or more of dipalmitoyl phosphatidylglycerole (DPPG), dioleoyl phosphatidylglycerole (DOPG), dimyristoyl phosphatidylglycerole (DMPG) and distearoyl phosphatidylglycerole (DSPG); and the glyceride is selected from glycerol trioleate or tricaprylin.

The organic solvent is selected from trichloromethane, ethyl ether, n-hexane or other organic solvent immiscible with water, preferably, trichloromethane or ethyl ether.

The isoosmotic regulator is selected from 0.9% sodium chloride injection, 5% mannitol injection, sodium lactate ringer's injection or 5% glucose injection; and the isoosmotic regulator is used to regulate the osmotic pressure of the liposome suspension to make same reach an isotonic state. The sodium lactate ringer's injection is a mixed solution of sodium lactate, sodium chloride, potassium chloride and calcium chloride each having a fixed amount, which is a solution known in this technical field.

The pH regulator is an alkaline substance with the concentration of 0.1M-1M, preferably, natrium hydroxide, triethylamine, lysine, arginine or histidine.

In the novel local anesthetic analgesic sustained-release drug delivery system, the dosage of each of the ingredients has the following range:
5ml of internal aqueous phase in total, including 5mg-500mg of analgesic, 1ml-5ml of drug solvent and 5mg-500mg of drug solubilizer; 100ml of external aqueous phase in total, selected from an aqueous solution prepared from one or more of 0.01g-10g of saccharide, 0.1g-10g of ring-shaped organic acid, 0.1g-10g of organic base and 0.1g-10g of deflocculant; 5ml of oil phase in total, including 5mg-400mg of synthetic phospholipid, 0.5mg-250mg of synthetic phosphatidylglycerol, 2.5mg-250mg of cholesterol and 2.5mg-250mg of glyceride; and the organic solvent, selected from one or more of 0.5ml-50ml of trichloromethane, 0.5ml-50ml of ethyl ether and 0.5ml-50ml of n-hexane.

Preferably, in the novel local anesthetic analgesic sustained-release drug delivery system, the dosage of each of the ingredients has the following range:
5ml of internal aqueous phase in total, including 25mg-300mg of analgesic, 1ml-5ml of drug solvent and 25mg-250mg of drug solubilizer; 100ml of external aqueous phase in total, selected from an aqueous solution prepared from one or more of 1g-5g of saccharide, 1g-5g of ring-shaped organic acid, 1g-3g of organic base and 1g-5g of deflocculant; 5ml of oil phase in total, including 25mg-150mg of synthetic phospholipid, 5mg-100mg of synthetic phosphatidylglycerol, 5mg-125mg of cholesterol and 1mg-150mg of glyceride; and the organic solvent, selected from one or more of 1.5ml-45ml of trichloromethane, 1.5ml-45ml of ethyl ether and 1.5ml-45ml of n-hexane.

For the novel local anesthetic analgesic sustained-release drug delivery system, the preparation method comprises the following steps:

### (1) Preparation of Internal Aqueous Phase

Weighing a certain amount of analgesic, and if the analgesic is in a free base form, adding a prescription amount of drug solvent and drug solubilizer to dissolve the analgesic completely; and if the analgesic is in an acid saline form, replacing the acid radical contained in the analgesic with an acid radical containing N or P, and then operating in accordance with the above-mentioned method.

### (2) Preparation of External Aqueous Phase

Weighing a certain amount of substances of an external aqueous phase, and adding water to dissolve same, wherein the organic base is used to regulate the pH value of the external aqueous phase.

### (3) Preparation of Oil Phase

Weighing a prescription amount of synthetic phosphatidylcholine, synthetic phosphatidylglycerol, cholesterol and glyceride, and dissolving same using an organic solvent to obtain an oil phase.

### (4) Preparation of Primary Emulsion

Adding the prepared internal aqueous phase into the oil phase with the volume ratio of 1:10-10:1, and shearing for 5-20min at 10000-16000rpm to obtain primary emulsion.

### 5) Preparation of Multiple Emulsion

Weighing a certain amount of primary emulsion, adding the primary emulsion into the external aqueous phase in accordance with the phase volume ratio of 1:5-1:50 of the primary emulsion to the external aqueous phase, shearing for 5-60s at 1000-4000rpm, then sequentially adding external aqueous phase which is 5-20 times the volume of the primary emulsion rapidly, blowing for 5-30min using 40-90L/min of nitrogen at 20-40°C or performing rotary evaporation at 20-40°C; and removing the organic solvent, collecting the intermediate of the liposome, centrifuging for 10-30min at 100-20000rpm, discarding the supernatant, flushing using a large number of isoosmotic regulator, regulating the pH to 5.0-8.0 using the pH regulator, and obtaining a multivesicular liposome, the grain size of the obtained multivesicular liposome ranging from 1µm to 50µm.

On the premise of conforming to the basic idea in this field, preferable multivesicular liposome preparations may be obtained through any combination of the above-mentioned technical features.

The present invention has the advantageous effect that: the present invention proposes conditions that must be met for preparing a multivesicular liposome as a specific drug, due to different drug structures, the required drug solubilizers are different, the structure of local anesthetics of amide derivatives contains benzene ring, pyridine ring, so that if ring-shaped organic acids are selected as drug solubilizers, the effect may be preferable, in addition, because the pyridine ring contains N atoms, if inorganic acids containing N and P in the same family are selected as drug solubilizers, a multivesicular liposome of high yield may be prepared. The multivesicular liposome prepared in the present invention has the advantages of high encapsulation percentage and drug loading capacity, uniform grain size, and good sustained-release effect. The multivesicular liposome of the present invention may be used to prepare drugs for treating chronic diseases that require long-term administration such as antidiabetic drugs, antidepressive drugs, drugs for treating cardiovascular diseases and the like.

### Description of Drawings

Fig. 1 is an appearance diagram of a levobupivacaine multivesicular liposome under a 400x optical microscope;
Fig. 2 is an appearance diagram of a levobupivacaine multivesicular liposome under a 200x optical microscope;
Figure 3 is a comparison diagram of mean acupuncture painless response number 48h after administration of levobupivacaine multivesicular liposome;
Figure 4 is a comparison diagram of mean acupuncture painless non-response number percentage (%) of to 48h after administration of levobupivacaine multivesicular liposome.

### Detailed Description

The present invention will be described below in detail in combination with drawings. Described embodiments are only used for explaining the present invention, but are not intended to limit the scope of the present invention.

### Embodiment 1

**Prescription Ingredients**

| | Type | Dosage |
|---|---|---|
| Oil Phase | DEPC | 30mg |
| | DOPC | 20mg |
| | Cholesterol | 25mg |
| | DPPG | 10mg |
| | Tricaprylin | 20mg |
| Organic Solvent | Trichloromethane | 3ml |
| | Ethyl Ether | 2ml |
| Internal Aqueous Phase 1 | Levobupivacaine Hydrochloride Injection | 160mg |
| | Phosphoric Acid (1M) | 3ml |
| | Dextrose | 150mg |
| | Vitamin C | 25mg |
| External Aqueous Phase | Nicotinic Acid | 500mg |
| | Arginine | Appropriate Amount |
| | Sodium Citrate | 1000mg |
| Isoosmotic Regulator | 0.9% Sodium Chloride | Appropriate Amount |
| pH Regulator | 1M Natrium Hydroxide | Appropriate Amount |

The preparation method of the multivesicular liposome is as follows:

### (1) Preparation of Internal Aqueous Phase

Weighing a certain amount of levobupivacaine hydrochloride, dissolving same in water and then adding 1M natrium hydroxide, filtering and collecting solid if there is no more insoluble substances, washing same with water for injection to neutral, drying at 60°C, adding other substances of the internal aqueous phase into the solid powder, adding 3ml of 1M phosphoric acid, dissolving same and then adding water to 5ml, to obtain an aqueous phase.

### (2) Preparation of External Aqueous Phase

Weighing the nicotinic acid and sodium citrate of the external aqueous phase in the above table, adding 80ml of water to dissolve same, regulating the pH to 7.4 using arginine, and adding water to 100ml, to obtain an external aqueous phase.

### (3) Preparation of Oil Phase

Weighing various substances of the oil phase in the above table, and dissolving same using 5ml of mixed solution of trichloromethane and ethyl ether (with the volume ratio of 3:2) to obtain an oil phase.

### (4) Preparation of Primary Emulsion

Adding the prepared internal aqueous phase into the oil phase, and shearing for 10min at 13000rpm to obtain primary emulsion.

### (5) Preparation of Multiple Emulsion

Weighing 5ml of primary emulsion, rapidly pouring 20ml of external aqueous phase, shearing for 60s at 3000rpm, sequentially adding 80ml of external aqueous phase rapidly, blowing for 20min using 80L/min of nitrogen, removing the organic solvent, collecting the intermediate of the multivesicular liposome, flushing using a large number of 0.9% NaCl solution, regulating the pH of the suspension of the liposome to 7.4 using 1M natrium hydroxide, and obtaining the multivesicular liposome, the mean grain size of the obtained multivesicular liposome being 30µm.

### Embodiment 2

**Prescription Ingredients**

| | Type | Dosage |
|---|---|---|
| Oil Phase | DEPC | 60mg |
| | Cholesterol | 40mg |
| | DPPG | 5mg |
| | Tricaprylin | 30mg |
| Organic Solvent | Trichloromethane | 4ml |
| | Ethyl Ether | 1ml |
| Internal Aqueous Phase | Bupivacaine | 200mg |
| | Nitric Acid (1M) | 2.5ml |
| | Cane Sugar | 200mg |
| | Vitamin C | 15mg |
| External Aqueous Phase | Glucuronic Acid | 100mg |
| | Arginine | Appropriate Amount |
| | Sodium Citrate | 1500mg |
| | Cane Sugar | 50mg |
| Isoosmotic | 0.9% Sodium Chloride | Appropriate Amount |
| pH Regulator | 0.8M Triethylamine | Appropriate Amount |

The preparation method of the multivesicular liposome is as follows:

### (1) Preparation of Internal Aqueous Phase

Weighing 200mg of bupivacaine, adding other substances of the internal aqueous phase, adding 2.5ml of 1M nitric acid, dissolving same and then adding water to 5ml, to obtain an aqueous phase.

### (2) Preparation of External Aqueous Phase

Weighing the glucuronic acid, cane sugar and sodium citrate of the external aqueous phase in the above table, adding 80ml of water to dissolve same, regulating the pH to 8.0 using arginine, and adding water to 100ml, to obtain an external aqueous phase.

### (3) Preparation of Oil Phase

Weighing various substances of the oil phase in the above table, and dissolving same using 5ml of mixed solution of trichloromethane and ethyl ether (with the volume ratio of 4:1) to obtain an oil phase.

### (4) Preparation of Primary Emulsion

Adding the prepared internal aqueous phase into the oil phase, and shearing for 8min at 16000rpm to obtain primary emulsion.

### 5) Preparation of Multiple Emulsion

Weighing 5ml of primary emulsion, rapidly pouring 20ml of external aqueous phase, shearing for 30s at 2000rpm, sequentially adding 80ml of external aqueous phase rapidly, performing rotary evaporation at 35 °C, removing the organic solvent, collecting the intermediate of the multivesicular liposome, flushing using a large number of 5% dextrose solution, regulating the pH of the suspension of the liposome to 6.8 using 0.8M triethylamine, and obtaining the multivesicular liposome, the mean grain size of the obtained multivesicular liposome being 43µm.

### Embodiment 3

**Prescription Ingredients**

| | Type | Dosage (mg/ml) |
|---|---|---|
| Oil Phase | DEPC | 30mg |
| | DSPC | 10mg |
| | Cholesterol | 15mg |
| | DSPG | 3mg |
| | Glycerol Trioleate | 15mg |
| Organic Solvent | Trichloromethane | 5ml |
| Internal Aqueous Phase | Ropivacaine | 250mg |
| | Phosphoric Acid (1M) | 3.5ml |
| | Glucuronic Acid | 100mg |
| External Aqueous Phase | Vitamin C | 50 |
| | Lysine | Appropriate Amount |
| | Sodium Tartrate | 50 |
| | Dextrose | 20 |
| Isoosmotic | Mannitol | 5% |
| pH Regulator | 0.2M Lysine | Appropriate Amount |

The preparation method of the multivesicular liposome is as follows:

### (1) Preparation of Internal Aqueous Phase

Weighing 250mg of ropivacaine, adding other substances of the internal aqueous phase, adding 3.5ml of 1M phosphoric acid, dissolving same and then adding water to 5ml, to obtain an aqueous phase.

### (2) Preparation of External Aqueous Phase

Weighing the vitamin C, cane sugar and sodium tartrate of the external aqueous phase in the above table, adding 80ml of water to dissolve same, regulating the pH to 8.5 using lysine, and adding water to 100ml, to obtain an external aqueous phase.

### (3) Preparation of Oil Phase

Weighing various substances of the oil phase in the above table, and dissolving same using 5ml of trichloromethane to obtain an oil phase.

### (4) Preparation of Primary Emulsion

Adding the prepared internal aqueous phase to the oil phase, and shearing for 10min at 13000rpm to obtain primary emulsion.

### (5) Preparation of Multiple Emulsion

Weighing 5ml of primary emulsion, rapidly pouring 20ml of external aqueous phase, shearing for 15s at 3000rpm, sequentially adding 80ml of external aqueous phase rapidly, performing rotary evaporation at 37 °C, removing the organic solvent, collecting the intermediate of the multivesicular liposome, flushing using a large number of 5% mannitol solution, regulating the pH of the suspension of the liposome to 7.2 using 0.2M lysine, and obtaining the multivesicular liposome, the mean grain size of the obtained multivesicular liposome being 24µm.

### Embodiment 4

**Prescription Ingredients**

| | Type | Dosage |
|---|---|---|
| Oil Phase | DOPC | 55mg |
| | Cholesterol | 30mg |
| | DOPG | 15mg |
| | Glycerol Trioleate | 10mg |
| Organic Solvent | Trichloromethane | 1ml |
| | Ethyl Ether | 4ml |
| Internal Aqueous Phase | Lidocaine | 300mg |
| | Nitric Acid (1M) | 4ml |
| | Vitamin C | 50mg |
| | Nicotinic Acid | 30mg |
| External Aqueous Phase | Vitamin C | 300mg |
| | Glucuronic Acid | 200mg |
| | Histidine | Appropriate Amount |
| | Sodium Dihydrogen | 400mg |
| | Dextrose | 2000mg |
| Isoosmotic | Sodium Lactate Ringer's | Appropriate Amount |
| pH Regulator | 0.5M Arginine | Appropriate Amount |

The preparation method of the multivesicular liposome is as follows:

### (1) Preparation of Internal Aqueous Phase

Weighing 300mg of lidocaine, adding other substances of the internal aqueous phase, adding 4ml of 1M nitric acid, dissolving same and then adding water to 5ml, to obtain an aqueous phase.

### (2) Preparation of External Aqueous Phase

Weighing the vitamin C, glucuronic acid, sodium dihydrogen phosphate and dextrose of the external aqueous phase in the above table, adding 80ml of water to dissolve same, regulating the pH to 9.0 using histidine, and adding water to 100ml, to obtain an external aqueous phase.

### (3) Preparation of Oil Phase

Weighing various substances of the oil phase in the above table, and dissolving same using 5ml of mixed solution of trichloromethane and ethyl ether (with the volume ratio of 1:4) to obtain an oil phase.

### (4) Preparation of Primary Emulsion

Adding the prepared internal aqueous phase into the oil phase, and shearing for 10min at 10000rpm to obtain primary emulsion.

### (5) Preparation of Multiple Emulsion

Weighing 5ml of primary emulsion, rapidly pouring 20ml of external aqueous phase, shearing for 15s at 4000rpm, sequentially adding 80ml of external aqueous phase rapidly, water bathing at 35°C, blowing for 15min using 100L/min of nitrogen, removing the organic solvent, collecting the intermediate of the multivesicular liposome, flushing using a large number of sodium lactate ringer's solution, regulating the pH of the suspension of the liposome to 7.4 using 0.5M arginine, and obtaining the multivesicular liposome, the mean grain size of the obtained multivesicular liposome being 45µm.

### Embodiment 5

**Prescription Ingredients**

| | Type | Dosage |
|---|---|---|
| Oil Phase | DMPC | 80mg |
| | Cholesterol | 50mg |
| | DMPG | 3mg |
| | Tricaprylin | 30mg |
| Organic Solvent | N-hexane | 3ml |
| | Ethyl Ether | 2ml |
| Internal Aqueous Phase | Mepivacaine | 180mg |
| | Phosphoric Acid (1M) | 3ml |
| | Vitamin C | 50mg |
| External Aqueous Phase | Glucuronic Acid | 600mg |
| | Lysine | Appropriate Amount |
| | Disodium Hydrogen | 500mg |
| Isoosmotic | 5% Dextrose | Appropriate Amount |
| pH Regulator | 0.2M Histidine | Appropriate Amount |

The preparation method of the multivesicular liposome is as follows:

### (1) Preparation of Internal Aqueous Phase

Weighing 180mg of mepivacaine, adding other substances of the internal aqueous phase, adding 3ml of 1M phosphoric acid, dissolving same and then adding water to 5ml, to obtain an aqueous phase.

### (2) Preparation of External Aqueous Phase

Weighing the glucuronic acid and disodium hydrogen phosphate of the external aqueous phase in the above table, adding 100ml of water to dissolve same, regulating the pH to 7.0 using lysine, adding water to 150ml, to obtain an external aqueous phase.

### (3) Preparation of Oil Phase

Weighing various substances of the oil phase in the above table, dissolving same using 5ml of mixed solution of n-hexane and ethyl ether (with the volume ratio of 3:2) to obtain an oil phase.

### (4) Preparation of Primary Emulsion

Adding the prepared internal aqueous phase into the oil phase, and shearing for 20min at 16000rpm to obtain primary emulsion.

### (5) Preparation of Multiple Emulsion

Weighing 5ml of primary emulsion, rapidly pouring 30ml of external aqueous phase, shearing for 60s at 4000rpm, sequentially adding 120ml of external aqueous phase rapidly, water bathing at 30°C, performing rotary evaporation, removing the organic solvent, collecting the intermediate of the multivesicular liposome, flushing using a large number of 5% dextrose solution, and regulating the pH of the suspension of the liposome to 7.4 using 0.2M histidine, and obtaining the multivesicular liposome, the mean grain size of the obtained multivesicular liposome being 15µm.

### Embodiment 6

**Prescription Ingredients**

| | Type | Dosage |
|---|---|---|
| Oil Phase | DEPC | 30mg |
| | DMPC | 25mg |
| | Cholesterol | 11mg |
| | DPPG | 0.5mg |
| | DSPG | 0.8mg |
| | Tricaprylin | 6mg |
| Organic Solvent | N-hexane | 3ml |
| | Trichloromethane | 2ml |
| Internal Aqueous Phase | Levobupivacaine | 220mg |
| | Nitric Acid (1M) | 2ml |
| | Vitamin C | 100mg |
| External Aqueous Phase | Vitamin C | 800mg |
| | Arginine | Appropriate Amount |
| | disodium hydrogen | 600mg for each |
| | Cane Sugar | 100mg |
| Isoosmotic | 0.9% Sodium Chloride | Appropriate Amount |
| pH Regulator | 0.1M Natrium Hydroxide | Appropriate Amount |

The preparation method of the multivesicular liposome is as follows:

### (1) Preparation of Internal Aqueous Phase

Weighing 220mg of levobupivacaine, adding other substances of the internal aqueous phase, adding 2ml of 1M phosphoric acid, dissolving same and then adding water to 5ml, to obtain an aqueous phase.

### (2) Preparation of External Aqueous Phase

Weighing the vitamin C, cane sugar, disodium hydrogen phosphate and sodium dihydrogen phosphate of the external aqueous phase in the above table, adding 100ml of water to dissolve same, regulating the pH to 7.0 using arginine, and adding water to 150ml, to obtain an external aqueous phase.

### (3) Preparation of Oil Phase

Weighing various substances of the oil phase in the above table, and dissolving same using 5ml of mixed solution of n-hexane and trichloromethane (with the volume ratio of 3:2) to obtain an oil phase.

### (4) Preparation of Primary Emulsion

Adding the prepared internal aqueous phase into the oil phase, and shearing for 10min at 15000rpm to obtain primary emulsion.

### 5) Preparation of Multiple Emulsion

Weighing 5ml of primary emulsion, rapidly pouring 30ml of external aqueous phase, shearing for 25s at 8000rpm, sequentially adding 120ml of external aqueous phase rapidly, water bathing at 35°C, performing rotary evaporation, removing the organic solvent, collecting the intermediate of the multivesicular liposome, flushing using a large number of 0.9% sodium chloride solution, regulating the pH of the suspension of the liposome to 7.0 using 0.1M natrium hydroxide, and obtaining the multivesicular liposome, the mean grain size of the obtained multivesicular liposome being 18µm.

### Embodiment 7

Microstructure Observation of Levobupivacaine Multivesicular Liposome

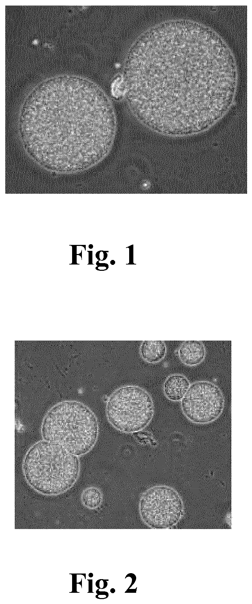

The levobupivacaine multivesicular liposome is prepared in accordance with the above-mentioned embodiment 1: take a drop of multivesicular liposome on the glass slide, cover the cover slip, observe under 400x microscope and 200x microscope, and shoot the microstructure diagram of the levobupivacaine multivesicular liposome, as shown in Fig. 1 and Fig. 2. The atlas shows that the multivesicular liposome is rounding spheroid in shape, and comprises multiple small vesicles therein. The envelope is complete and clearly visible, and the entire microstructure grain size is uniform.

### Embodiment 8

Determination of Content and Encapsulation Percentage of Levobupivacaine Multivesicular Liposome

The content of the levobupivacaine multivesicular liposome is determined using high performance liquid chromatography, wherein the chromatographic conditions are as follows:

Chromatographic column: Agilent C18 column (150mm^{∗}4.6mm^{∗}5µm); The mixed solution of 0.02mol/L phosphate buffered saline (weighing 2.72g of potassium dihydrogen phosphate and 0.75g of natrium hydroxide, adding 1000ml of water to dissolve same, and regulating the pH value to 8.0)-acetonitrile (50:50) is used as a mobile phase; the detection wavelength is 240nm; the flow velocity is 1.0ml/min; the column temperature is 35°C, and the injection volume is 20µl. The resolution between the levobupivacaine peak and an impurity peak adjacent thereto should be greater than 1.5.

The specific method for determination of the encapsulation percentage of the levobupivacaine multivesicular liposome is as follows:
precisely measuring 1.0ml of suspension of levobupivacaine multivesicular liposome, adding 1.0ml of 0.9% NaCl solution, precisely transferring 0.1ml after blending, placing same in 10ml of volumetric flask, adding 2ml of methanol, performing demulsification and vibration and diluting same to the scale using the mobile phase, and shaking well; determining the total dosage (Wₜₒₜₐₗ) in the levobupivacaine multivesicular liposome using HPLC; centrifuging other samples for 10min at 3000rpm, precisely transferring 0.1ml of supernatant, placing same in 10ml of volumetric flask, adding 2ml of methanol to perform demulsification and vibration, diluting same to the scale using the mobile phase, shaking well, sucking 20µl, operating in accordance with the above-mentioned chromatographic condition, and determining the content of free drug in the levobupivacaine multivesicular liposome (Wₛᵤₚₑᵣₙₐₜₐₙₜ).

The encapsulation percentage (EE%) of the levobupivacaine multivesicular liposome is computed in accordance with the formula: encapsulation percentage (EE%) = (Wₜₒₜₐₗ - Wₛᵤₚₑᵣₙₐₜₐₙₜ)/Wₜₒₜₐₗ × 100%. The encapsulation percentage of the multivesicular liposome in the embodiment is determined in accordance with the above-mentioned method, see the following table:

| Embodiments | Encapsulation Percentage (%) |
|---|---|
| Embodiment 1 | 85 |
| Embodiment 2 | 91 |
| Embodiment 3 | 93 |
| Embodiment 4 | 90 |
| Embodiment 5 | 85 |
| Embodiment 6 | 82 |

### Embodiment 9

Pesticide Effect Comparison of Levobupivacaine Multivesicular Liposome

30-week guinea pigs which are 300-500g in weight and are male are used as experimental animals. Before the experiment, the guinea pigs should have a rest-cure for 3 days in an independent environment at the room temperature of (23±1)°C under 12-12h bright/dark illumination (illumination is at 8:00 a.m) to adapt to the environment.

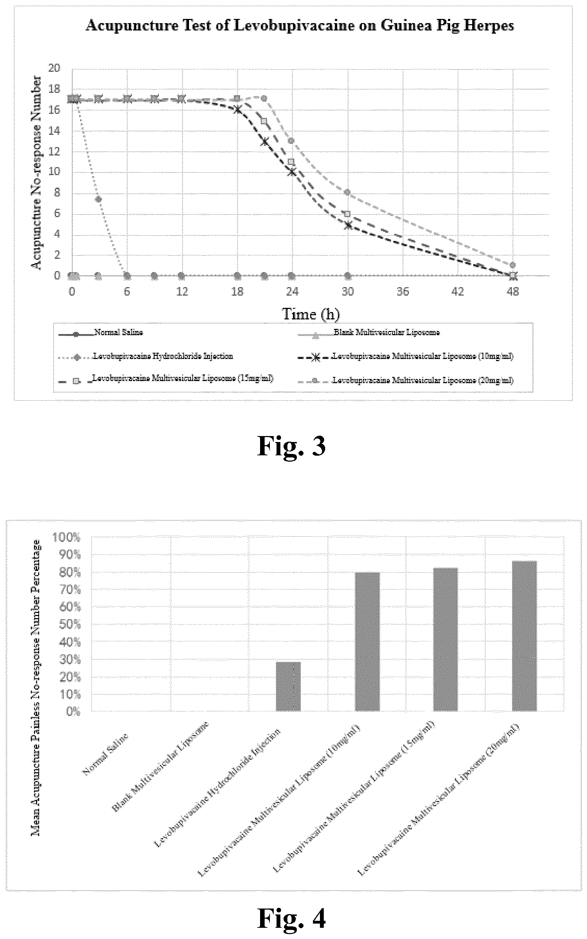

Experimental method: the hair on 6cm × 10cm of back skin of guinea pigs (nine) is completely shaved. The herpes range is marked, and 4 regions are marked for each guinea pig, wherein the upper left region indicates normal saline, the upper right region indicates blank multivesicular liposome, the left lower region indicates levobupivacaine hydrochloride injection, and the lower right region indicates self-control levobupivacaine multivesicular liposome. The experiment is divided into three dosage groups, i.e. low dosage group 10mg/ml, middle dosage group 15mg/ml, and high dosage group 20mg/ml, each group including three guinea pigs. The injection volume of intradermal injection for administration of each marked region is 0.35ml. Subcutaneous herpes is formed, and the response of the guinea pigs to acupuncture is tested after 15min. Each herpes is acupunctured for 17 times, wherein the acupuncture interval is 3-5s, and the test time point are respectively: 1min, 15min, 30min, 3h, 6h, 9h, 12h, 18h, 21h, 24h, 30h and 48h after injection, 12 time points in total. Each guinea pig is acupunctured for 204 times in total in each region, and the acupuncture to which the guinea pigs cannot respond is counted. The number of acupuncture not making a response at the 12 time points within 48h is accumulated, and the degree of anesthesia is reflected through the value formed by taking the sum as a numerator and the total acupuncture number 204 as a denominator. The mean percentage of number of times of non-response of each group of guinea pigs is computed, the bigger the value is, the stronger the anesthetic effect is; the smaller the computation value is, the weaker the anesthetic effect is. See Table 1 and Figure 2 for result of mean acupuncture number 48h after administration of levobupivacaine multivesicular liposome in low dosage group, middle dosage group and high dosage group at different time points. See Table 2 and Figure 3 for result of mean acupuncture painless response number percentage (%) of.

**Table 1: Statistical Table of Mean Acupuncture Number of Levobupivacaine Multivesicular Liposome at Different Time Points**

| Experimental Group | 1min | 15min | 30min | 3h | 6h | 9h | 12h | 18h | 21h | 24h | 30h | 48h |
|---|---|---|---|---|---|---|---|---|---|---|---|---|
| Normal Saline | 0 | 0 | 0 | 0 | 0 | 0 | 0 | 0 | 0 | 0 | 0 | 0 |
| Blank Multivesicular Liposome | 0 | 0 | 0 | 0 | 0 | 0 | 0 | 0 | 0 | 0 | 0 | 0 |
| Levobupivacaine Hydrochloride Injection | 17 | 17 | 17 | 7.33 | 0 | 0 | 0 | 0 | 0 | 0 | 0 | 0 |
| Levobupivacaine Multivesicular Liposome (10mg/ml) | 17 | 17 | 17 | 17 | 17 | 17 | 17 | 16 | 13 | 10 | 5 | 0 |
| Levobupivacaine Multivesicular Liposome (15mg/ml) | 17 | 17 | 17 | 17 | 17 | 17 | 17 | 17 | 15 | 11 | 6 | 0 |
| Levobupivacaine Multivesicular Liposome (20mg/ml) | 17 | 17 | 17 | 17 | 17 | 17 | 17 | 17 | 17 | 13 | 8 | 1 |

**Table 2: Result of Mean Painless Response Number Percentage (%) of Levobupivacaine Multivesicular Liposome at Different Time Points**

| Experimental Group | Normal Saline | Blank Multivesic ular Liposome | Levobupiva caine Hydrochlori de Injection | Levobupivacaine Multivesicular Liposome (10mg/ml) | Levobupivaca ine Multivesicular Liposome (15mg/ml) | Levobupiva caine Multivesicular Liposome (20mg/ml) |
|---|---|---|---|---|---|---|
| Mean Painless Response Number | 0 | 0 | 0.2859 | 0.7990 | 0.8235 | 0.8578 |
| Mean Painless Response Number Percentage (%) | 0 | 0 | 28.59 | 79.9 | 82.35 | 85.78 |

The pharmacodynamic experiment of the levobupivacaine multivesicular liposome demonstrates that the acupuncture painless response number (10mg/ml: 79.90%; 13.3mg/ml: 82.35%; 20mg/ml: 85.78% respectively) of the levobupivacaine multivesicular liposome within 48 hours is apparently higher than that of levobupivacaine hydrochloride injection (28.59%), and the action duration is greater than 24 hours. Therefore, it can be implemented that levobupivacaine is prepared into multivesicular liposome suspension with a sustained release function and then is used for postoperative analgesia.

The above is just preferred embodiments of the present invention and is not intended to limit the present invention. Any modification, equivalent replacement, improvement, etc. made within the spirit and the principle of the present invention shall be contained within the protection scope of the present invention.

## Claims

1. A novel local anesthetic analgesic sustained-release drug delivery system, including an internal aqueous phase, an external aqueous phase, an oil phase, an organic solvent, an isoosmotic regulator and a pH regulator, wherein the internal aqueous phase includes an analgesic, a drug solvent and a drug solubilizer, wherein the analgesic is selected from one of bupivacaine, levobupivacaine, ropivacaine, lidocaine and mepivacaine, and the analgesic is in a free base form or an acid saline form; the drug solvent is selected from inorganic acid containing N or P; and the drug solubilizer is selected from one or more of saccharide and ring-shaped organic acid.

2. The novel local anesthetic analgesic sustained-release drug delivery system of claim 1, wherein the external aqueous phase is selected from an aqueous solution prepared from one or more of saccharide, ring-shaped organic acid, organic base and deflocculant; the oil phase includes synthetic phospholipid, synthetic phosphatidylglycerol, cholesterol and glyceride; the organic solvent is selected from one or more of trichloromethane, n-hexane and ethyl ether; the isoosmotic regulator is selected from 0.9% sodium chloride injection, 5% mannitol injection, sodium lactate ringer's injection or 5% glucose injection; and the pH regulator is selected from sodium hydroxide, triethylamine, lysine, arginine or histidine.

3. The novel local anesthetic analgesic sustained-release drug delivery system of claim 2, wherein the saccharide is selected from dextrose, fructose or cane sugar; the ring-shaped organic acid is selected from vitamin C, nicotinic acid, gallic acid or glucuronic acid; the organic base is selected from lysine, arginine or histidine; the deflocculant is selected from citrate, tartrate or phosphate; the synthetic phospholipid is selected from one or more of DEPC, DOPC, DPPC, DSPC and DMPC; the synthetic phosphatidylglycerol is selected from one or more of DPPG, DOPG, DMPG and DSPG; and the glyceride is selected from glycerol trioleate or tricaprylin.

4. The novel local anesthetic analgesic sustained-release drug delivery system of claim 2, wherein the dosage of each of the ingredients has the following range: 5ml of internal aqueous phase in total, including 5mg-500mg of analgesic, 1ml-5ml of drug solvent and 5mg-500mg of drug solubilizer; 100ml of external aqueous phase in total, selected from one or more of 0.01g-10g of saccharide, 0.1g-10g of ring-shaped organic acid, 0.1g-10g of organic base and 0.1g-10g of deflocculant; 5ml of oil phase in total, including 5mg-400mg of synthetic phospholipid, 0.5mg-250mg of synthetic phosphatidylglycerol, 2.5mg-250mg of cholesterol and 2.5mg-250mg of glyceride; and the organic solvent, selected from one or more of 0.5ml-50ml of trichloromethane, 0.5ml-50ml of ethyl ether and 0.5ml-50ml of n-hexane.

5. The novel local anesthetic analgesic sustained-release drug delivery system of claim 4, wherein the dosage of each of the ingredients has the following range: 5ml of internal aqueous phase in total, including 25mg-300mg of analgesic, 1ml-5ml of drug solvent and 25mg-250mg of drug solubilizer; 100ml of external aqueous phase in total, selected from an aqueous solution prepared from one or more of 1g-5g of saccharide, 1g-5g of ring-shaped organic acid, 1g-5g of organic base and 1g-5g of deflocculant; 5ml of oil phase in total, including 25mg-150mg of synthetic phospholipid, 5mg-100mg of synthetic phosphatidylglycerol, 5mg-125mg of cholesterol and 1mg-150mg of glyceride; and the organic solvent, selected from one or more of 1.5ml-45ml of trichloromethane, 1.5ml-45ml of ethyl ether and 1.5ml-45ml of n-hexane.

6. The novel local anesthetic analgesic sustained-release drug delivery system of claim 4 or 5, wherein the preparation method comprises the following steps:
(1) preparation of internal aqueous phase
weighing a certain amount of analgesic, and if the analgesic is in a free base form, adding a prescription amount of drug solvent and drug solubilizer to dissolve the analgesic completely; and if the analgesic is in an acid saline form, replacing the acid radical contained in the analgesic with an acid radical containing N or P, and then operating in accordance with the above-mentioned method;
(2) preparation of external aqueous phase
weighing a certain amount of substances of an external aqueous phase, and adding water to dissolve same, wherein the organic base is used to regulate the pH value of the external aqueous phase;
(3) preparation of oil phase
weighing a prescription amount of synthetic phosphatidylcholine, synthetic phosphatidylglycerol, cholesterol and glyceride, and dissolving same using an organic solvent to obtain an oil phase;
(4) preparation of primary emulsion
adding the prepared internal aqueous phase into the oil phase with the volume ratio of 1:10-10:1, and shearing for 5-20min at 10000-16000rpm to obtain primary emulsion;
(5) preparation of multiple emulsion
weighing a certain amount of primary emulsion, adding the primary emulsion into the external aqueous phase in accordance with the phase volume ratio of 1:5-1:50 of the primary emulsion to the external aqueous phase, shearing for 5-60s at 1000-4000rpm, then sequentially adding external aqueous phase which is 5-20 times the volume of the primary emulsion rapidly, blowing for 5-30min using 40-90L/min of nitrogen at 20-40°C or performing rotary evaporation at 20-40°C; and removing the organic solvent, collecting the intermediate of the multivesicular liposome, centrifuging for 10-30min at 100-20000rpm, discarding the supernatant, flushing using the isoosmotic regulator, regulating the pH to 5.0-8.0 using the pH regulator, and obtaining a multivesicular liposome, the grain size of the obtained multivesicular liposome ranging from 1µm to 50µm.

7. The novel local anesthetic analgesic sustained-release drug delivery system of claim 1, wherein the administration route thereof is local injection administration.

8. The novel local anesthetic analgesic sustained-release drug delivery system of claim 1, wherein the system is used for pre-operation anesthesia and post-operation analgesia.
